# EUROPÄISCHE PATENTSCHRIFT

(11) **EP 1 740 106 B1**
(45) Veröffentlichungstag und Bekanntmachung des Hinweises auf die Patenterteilung: **23.09.2009**
(21) Anmeldenummer: 05740230.7
(22) Anmeldetag: 15.04.2005
(51) Int. Cl.: A61B 17/32

(54) **APPLIKATOR FÜR DIE WASSERSTRAHL-CHIRURGIE**
APPLICATOR FOR WATER JET SURGERY
APPLICATEUR POUR LA CHIRURGIE A JETS D'EAU

(30) Priorität: 28.04.2004 DE 102004020855
(43) Veröffentlichungstag der Anmeldung: 10.01.2007
(73) Patentinhaber: Erbe Elektromedizin GmbH, 72072 Tübingen (DE)
(72) Erfinder: KLEIN, Ralf, 72108 Rottenburg (DE); QUECK, Jochen, 72076 Tübingen (DE); FISCHER, Klaus, 72202 Nagold (DE)
(74) Vertreter: Bohnenberger, Johannes
(86) Internationale Anmeldenummer: PCT/EP2005/004045
(87) Internationale Veröffentlichungsnummer: WO 2005/104965

(56) Entgegenhaltungen:
- WO-A-96/39954
- US-A- 5 853 384
- US-B1- 6 216 573

## Beschreibung

Die Erfindung betrifft einen Applikator für die Wasserstrahl-Chirurgie nach dem Oberbegriff des Patentanspruches 1.

Für die Dissektion von Parenchymgewebe wird in zunehmendem Maße die Wasserstrahl-Chirurgie verwendet. Hierfür ist beispielsweise aus der US 6,216,573 ein Gerät mit einem Handgriff bekannt, das eine distal angeordnete Düse aufweist, die über eine Kupplung mit einem Druckschlauch verbunden ist, über welchen ein Arbeitsfluid, insbesondere eine Ringerlösung, von einer Druckquelle mit einem sehr hohen Druck zugeführt werden kann. Aufgrund des hier verwendeten extrem hohen Arbeitsdruckes (bis zu mehreren Hundert bar) ist das Ankoppeln sehr problematisch.

Weiterhin sind Wasserstrahl-Chirurgiegeräte bekannt, bei welchen die Druckleitung von der Druckquelle bis zur Düse reicht, so daß keine gesonderte Kupplung notwendig ist. Nachdem eine Sterilisation des Druckschlauches nach Benutzung nicht möglich ist, sind Operationen mit einem derartigen Gerät insbesondere dann sehr teuer, wenn während der Operation Geräte mit verschiedenen Applikatoren verwendet werden müssen, um schwer zugängliche Stellen behandeln zu können. Dokument WO-A-96/39954 offenbart einen Applikator für die Wasserstrahlchirurgie, umfassend einen Handgriff, eine Druckleitung mit einer Düse am Ende der Leitung, wobei die Druckleitung am distalen Ende verformbar ausgebildet ist.

Ausgehend vom oben genannten Stand der Technik ist es Aufgabe der vorliegenden Erfindung, einen Applikator für die Wasserstrahl-Chirurgie aufzuzeigen, der in einfacher und kostengünstiger Weise die Anwendungsbreite des Applikators erhöht.

Diese Aufgabe wird bei einem Applikator für die Wasserstrahl-Chirurgie, umfassend einen Handgriff, eine Druckleitung zur Zuführung eines Arbeitsfluids aus einer Druckquelle und eine Düse am Ende der Druckleitung zur Erzeugung eines Fluidstrahls mit definierten Eigenschaften dadurch gelöst, daß ein Set von Formteilen mit voneinander verschiedenen Größen und/oder Formen vorgesehen ist, die mit dem Handgriff reversibel verbindbar sind und Führungseinrichtungen aufweisen, um die elastisch verformbar ausgebildete Druckleitung vom Handgriff bis zur Düse in einem durch das jeweilige Formteil vorbestimmten Verlauf zu führen.

Ein wesentlicher Gedanke der Erfindung besteht also darin, daß der Druckschlauch während einer Operation ständig an der Druckquelle angeschlossen bleibt und nur das Formteil gewechselt wird, um eine, dem momentanen Operationsschritt angepaßte Gestaltung des Applikators zu erreichen, die auf einfache Weise während der Operation durch Verwendung eines anderen Formteils unter Beibehaltung des Druckschlauches veränderbar ist.

Die Führungseinrichtung ist vorzugsweise derart ausgebildet, daß die am Handgriff befestigte Druckleitung bei einem Wechseln des Formteils aus der Führungseinrichtung herausnehmbar und wieder einsetzbar ist. Dadurch ist ein leichter Wechsel der Führungseinrichtung möglich.

Bei einer Ausführungsform der Erfindung sind die Formteile rohrförmig ausgebildet, so daß die Führungseinrichtungen das Lumen dieser Rohre umfassen. Diese Ausführungsform ist besonders leicht herstellbar.

Vorzugsweise sind Saugeinrichtungen vorgesehen, um Arbeitsflüssigkeit und auch Blut oder andere Körperflüssigkeiten abzusaugen. Dies ist dann besonders einfach, wenn die Formteile rohrförmig ausgebildet sind und das Lumen an eine Saugeinrichtung anschließbar ist.

Das Set von Formteilen umfaßt vorzugsweise mindestens ein geradliniges Formteil und ein gekrümmtes oder abgewinkeltes Formteil. Mit diesen beiden Ausführungsformen sind die meisten Anwendungen möglich. Vorteilhafterweise werden diese Formteile in verschiedenen Größen vorgehalten, so daß alle Operationsbedingungen abdeckbar sind.

Bei einer besonders bevorzugten Ausführungsform umfaßt das Formteil mindestens an seinem vom Handgriff abgewandten distalen Ende Elektrodeneinrichtungen, die mit einem HF-chirurgischen Generator zur Koagulation von Gewebe verbindbar sind. Mit dieser Ausführungsform wird somit ein weiteres, bei derartigen Operationen wesentliches Problem gelöst, das darin besteht, daß bisher zur Darstellung und Versorgung von Gefäßen oder Gallengängen (bei der Leber) Ligaturen, Clips oder Hochfrequenz-Strom verwendet werden, wobei jedes Mal ein Instrumentenwechsel notwendig ist. Der dafür notwendige Zeitaufwand ist für den Patienten ebenso wie für den durchführenden Operateur außerordentlich belastend. Durch die erfindungsgemäße Ausbildung des Applikators kann ein solcher Instrumentenwechsel erspart werden.

Derartige Elektrodeneinrichtungen können in verschiedener Weise ausgebildet sein. Zum einen besteht die Möglichkeit, durch einen direkten Kontakt zwischen der Elektrode und dem Gewebe einen Koagulationsstsom fließen zu lassen. Bei einer anderen bevorzugten Ausführungsform umfaßt das Formteil eine Einrichtung, insbesondere eine schlauchförmige Sonde zur Zuführung von Edelgas sowie eine vorzugsweise in der Sonde angebrachte Elektrode zur Zuführung eines HF-Stroms zur Bildung einer Edelgas-Plasma-Koagulationseinrichtung, wie sie an sich bekannt ist.

Das Formteil umfaßt weiterhin vorzugsweise eine bis zur Düse reichende Leitung zum Zu- und/oder Abführen von Fluiden, so daß ein Spülen und Absaugen im Operationsgebiet möglich ist. Die Saugleitung umfaßt vorzugsweise eine Saugöffnung, welche vorzugsweise in einer von der durch die Düse vorgegebenen Richtung abweicht. Dadurch ist ein gleichzeitiges Saugen und Applizieren des Wasserstrahls ohne dessen Störung möglich.

Vorzugsweise ist die Druckleitung mit Düse ggf. zusammen mit dem Handgriff zur einmaligen Verwendung bestimmt, während das Formteil sterilisierbar ausgebildet ist. Dadurch können die Operationskosten niedrig gehalten werden.

Das Formteil und/oder die Druckleitung umfassen vorzugsweise Justiereinrichtungen, die derart ausgebildet sind, daß die Düse in einer definierten Position zum Formteil positionierbar ist. Auf diese Weise wird die erzielbare Arbeitsgenauigkeit gesteigert.

Bei dieser Ausführungsform können die Justiereinrichtungen eine Andrückeinrichtung umfassen zum Andrücken der Druckleitung in Richtung des Fluidstrahls an einen am Formteil ausgebildeten Zentrieranschlag. Auf diese Weise wird nicht nur die Richtung, sondern auch die "Endposition" der Düse relativ zum Handgriff definiert. Die Druckleitung kann einen, die Düse beinhaltenden verformbaren Endabschnitt aufweisen, der mit einem zur Druckquelle führenden Zuführungsabschnitt über eine Kupplung verbindbar ist. Auf diese Weise kann besonders bei verschiedenen Längen der Formteile eine verbesserte Anpassung von Druckschlauch und Formteil erzielt werden. Hierbei ist es auch möglich, den Endabschnitt zusammen mit dem Formteil austauschbar zu gestalten, was das Auswechseln von Formteilen unter Umständen vereinfachen kann.

Nachfolgend werden bevorzugte Ausführungsformen der Erfindung anhand von Abbildungen näher erläutert. Hierbei zeigen
- Fig. 1: einen Handgriff mit Druckleitung,
- Fig. 2: eine Ausführungsfom eines Formteils,
- Fig. 3: einen Teil-Längsschnitt der Verbindungsstelle zwischen Formteil und Handgriff,
- Fig. 4-1 bis Fig. 4-5: verschiedene bevorzugte Formen von Formteilen,
- Fig. 5: einen Handgriff mit angesetztem Formteil,
- Fig. 6 bis 8: Schnitte durch den Bereich VI-VIII von Fig. 5 betreffend verschiedene Ausführungsformen,
- Fig. 9: einen Teillängsschnitt durch das zuführungsseitige Ende des Handgriffes,
- Fig. 10: eine weitere Ausführungsform eines Handgriffs in einer Darstellung gemäß Fig. 9,
- Fig. 11: eine weitere Ausführungsform der Erfindung in einer Darstellung entsprechend der nach Fig. 1,
- Fig. 12: ein Formteil zur Verwendung mit dem Handgriff nach Fig. 11,
- Fig. 13: eine vergrößerte Schnittdarstellung des Kopplungsbereiches, zwischen Handstück und Formteil nach den Figuren 11 und 12, und
- Fig. 14: eine schematisierte Darstellung eines weiteren Formteiles mit HF-Koagulations-Vorrichtungen.

In der nachfolgenden Beschreibung werden für gleiche und gleich wirkende Teile dieselben Bezugsziffern verwendet.

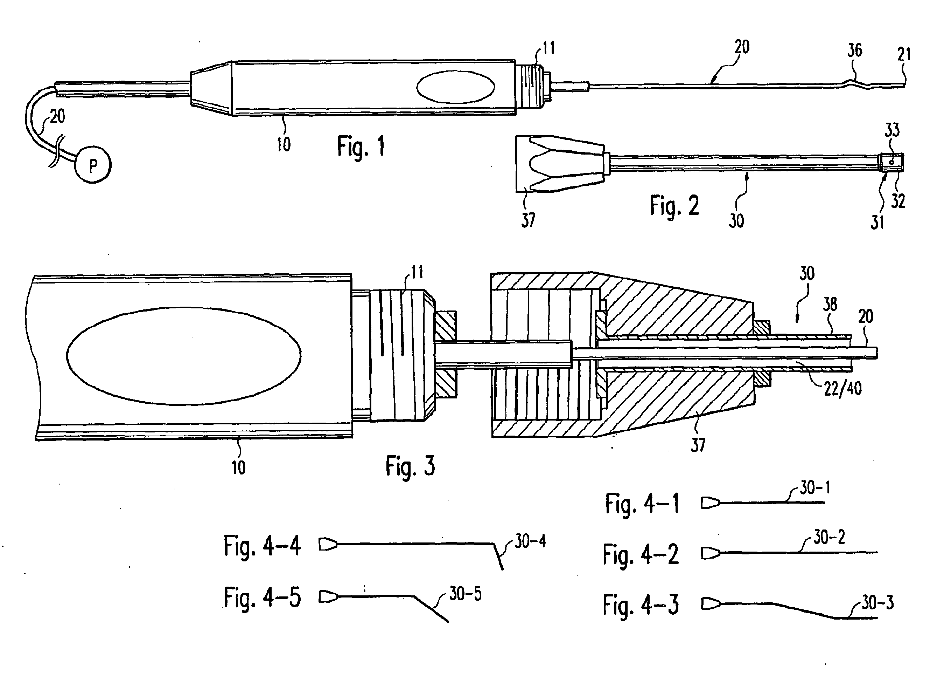

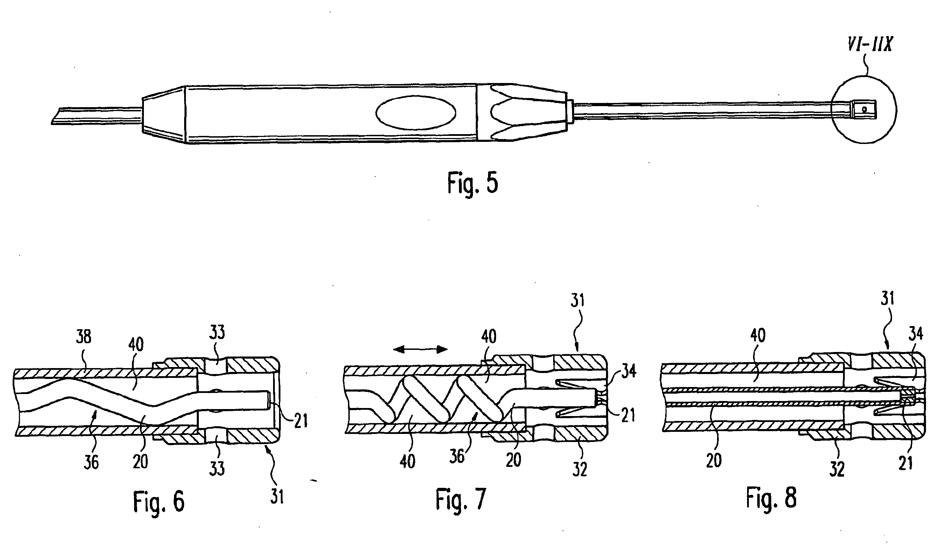

Wie aus den Figuren 1 bis 3 hervorgeht, umfaßt der Applikator einen Handgriff 10, durch den eine Druckleitung 20 von einer Druckquelle P bis zu einer Düse 21 geführt ist. Der Handgriff 10 weist an seinem distalen Ende ein Schraubgewinde 11 auf, über welches ein Formteil 30 mit einem entsprechend gestalteten Schraubgewinde in einer Überwurfkappe 37 befestigbar ist. Die Überwurfkappe 37 sitzt drehbeweglich, aber in Längsrichtung fixiert, auf einem Rohr 38. Durch das Lumen 40 des Rohres 38 ist die Druckleitung 20 geführt, die an ihrem distalen Ende bei der hier gezeigten Ausführungsform eine Zentrierwendel 36 aufweist. Diese Zentrierwendel oder welle ist, wie in den Figuren 6 und 7 gezeigt, so dimensioniert, daß das Ende der Druckleitung 20 fest im Lumen 40 des Rohres 38 gehalten ist und die Düse 21 im wesentlichen im Zentrum des Rohres 38 kurz vor dessen Ende positionierbar ist.

Am distalen Ende 31 des Rohres 38 bzw. des Formteils 30 ist ein Endstück 32 vorgesehen, das seitlich angebrachte Saugöffnungen 33 aufweist. Das Lumen 40 bildet in diesem Fall gleichzeitig eine Leitung 22, die mit einer Saugeinrichtung verbindbar ist.

Wie in den Fig. 4-1 bis 4-5 gezeigt, können die Formteile 30 in verschiedenen Längen (30-1; 30-2), und in verschiedenen Krümmungsformen (30-3 bis 30-5) und auch hier wieder in verschiedenen Längen vorgesehen sein. Die Form und Länge wird hierbei den Operationsanforderungen entsprechend gestaltet.

Zur Benutzung wird der Druckschlauch 20 an die Druckquelle angeschlossen. Der Operateur wählt nun dasjenige Formteil 30-1 bis 30-5, das er bei seinen ersten Operationsschritten verwenden will und setzt das Formteil 30 auf den Handgriff 10 auf und führt hierbei den Druckschlauch 20 durch das Lumen 40 des Rohres 38 in das Formteil 30 ein, schraubt die Überwurfkappe 37 auf dem Gewinde 11 des Handgriffes 10 fest, so daß die Düse 21 am distalen Ende 31 des Formteils 30 zu liegen kommt. Will der Operateur ein anderes Formteil, z.B. ein gekrümmtes Formteil gemäß Fig. 4-5 verwenden, so schraubt er das bisher verwendete Formteil 30-1 ab und ersetzt es durch ein Formteil 30-5 unter Vollzug der vorher beschriebenen Schritte. Nach dem Ende der Operation können die Formteile 30 sterilisiert und für eine neue Operation bereitgestellt werden, der Druckschlauch 20 wird ggf. zusammen mit dem Handgriff nicht mehr verwendet, da der Druckschlauch 20 wegen seines geringen Lumen- und Düsendurchmessers nicht sterilisierbar ist.

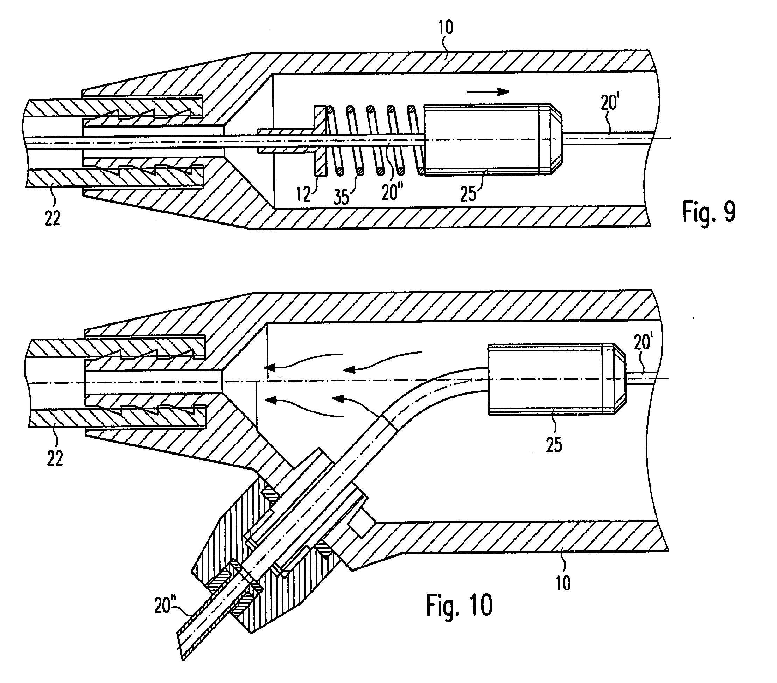

Zum Zentrieren der Düse 21 innerhalb des distalen Endes 31 des Formteils 30 kann, wie in den Figuren 7 und 8 gezeigt, im Endstück 32 ein Justieranschlag 34 derart angebracht sein, daß beim Zusammenbau von Handgriff 10 und Formteil 30 das Ende der Druckleitung 20 mit der Düse 21 in den Justieranschlag 34, der vorzugsweise konisch ausgebildet ist, hineingesteckt und dort in einer Endposition fixiert Wird, so daß die Lage der Düse 21 sowohl in Längsrichtung des Rohres 38 als auch in Radialrichtung des Lumens 40 exakt definiert ist. Längenunterschiede können entweder durch die Zentrierwendel 36 kompensiert werden oder - wie in Fig. 9 gezeigt - über eine Feder 35, welche sich im Handgriff auf einem Federsitz 12 abstützt und einen Endabschnitt 20' der Druckleitung 20 über einen Halter 25, der auf der Druckleitung 20 befestigt ist, in Richtung auf die Düse schiebt.

Der Halter 25 kann auch gleichzeitig als Kupplung ausgebildet sein, wie dies in Fig. 10 gezeigt ist, über welche ein dünnerer, distaler Endabschnitt 20' der Druckleitung 20 mit einem dickeren Zuführungsabschnitt 20" verbunden ist.

Die Ausführungsform des Handgriffes nach Fig. 10 unterscheidet sich weiterhin von der nach Fig. 9 dadurch, daß die Saugleitung 22 nicht den Druckschlauch 20 koaxial umgibt, sondern vielmehr als gesonderte Leitung ausgeführt ist, während der Druckschlauch 20 bzw. dessen Zuführungsabschnitt 20" durch eine weitere (abgedichtete) Öffnung im Handgriff 10 herausgeführt ist.

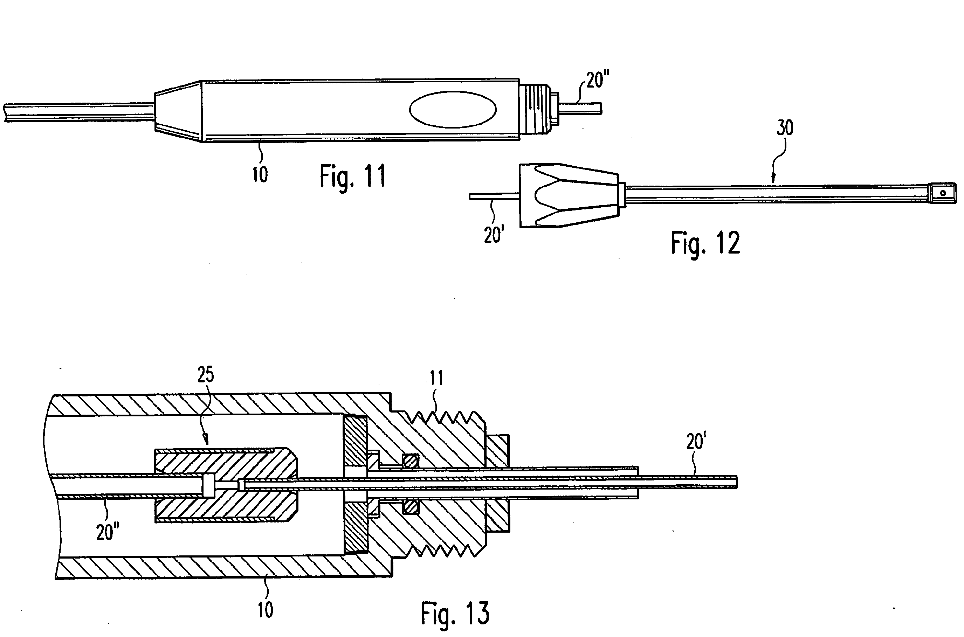

Die in den Figuren 11 bis 13 gezeigte Ausführungsform der Erfindung unterscheidet sich von der zuvor beschriebenen Ausführungsform dadurch, daß beim Auswechseln eines Formteils 30 zusammen mit diesem auch der Endabschnitt 20' der Druckleitung ausgewechselt wird. Hierfür ist im Handgriff 10 eine Kupplung 25 derart positioniert, daß beim Aufstecken und Aufschrauben des Formteils 30 das der Düse 21 gegenüberliegende Ende des Endabschnittes 20' in die im Handgriff 10 fixiert Kupplung 25 inseriert und durch die elastische Ausbildung der Kupplung 25 druckfest mit dem Zuführungsabschnitt 20" der Druckleitung 20 verbunden wird.

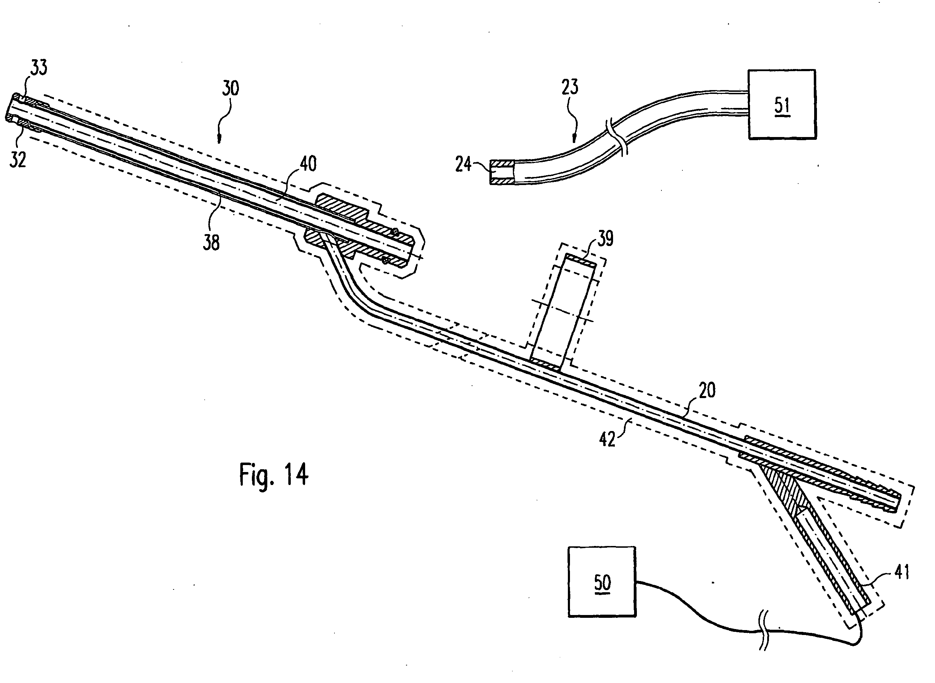

Die in Fig. 14 gezeigte Ausführungsform der Erfindung umfaßt ein Formteil 30, das eine Gewebekoagulation mit HF-Strom ermöglicht. Der Handgriff 10 mit daraus hervorstehendem Druckschlauch 20 wird wie mit den "einfachen" Formteilen 30-1 bis 30-4 (wobei hier anstelle der Gewindeverbindung eine einfachere Steckverbindung gezeigt ist) so verbunden, daß der Druckschlauch 20 mit der Düse 21 im Endstück 32 fixiert wird. Das Formteil 30 hat hierbei einen Halter 39, um dieses Formteil mit dem Handgriff zu verbinden.

Weiterhin weist das Formteil 30 einen HF-Anschluß 41 auf, der mit einem HF-Generator 50 verbindbar ist. Die Saugleitung 20 ist metallisch ausgebildet und mit dem ebenfalls metallischen Rohr 38 verbunden. Die Gesamtanordnung ist über eine Isolationsschicht 42 in seiner Gesamtheit nach außen hin elektrisch isoliert bis auf das (metallisch ausgebildete) Endstück 32, welches bei dieser Ausführungsform der Erfindung als Koagulationselektrode dient. Der Operateur kann also mit diesem Instrument gleichzeitig mittels des Wasserstrahls schneiden und bei Bedarf mittels der "Elektrode" 32 Gewebe koagulieren.

Alternativ oder auch zusätzlich kann eine Sonde 23 mit innenliegender Elektrode 24, die mit einer APC-Koagulationseinrichtung 51 verbunden sind, zusammen mit dem Druckschlauch 20 in das Lumen 40 eingeschoben werden. Der Justieranschlag 34 (in dieser Abbildung nicht gezeigt) wird dementsprechend verändert und so ausgebildet, daß sowohl die (an sich bekannte) Sonde 23 als auch der Druckschlauch 20 innerhalb des Endstückes 32 exakt reproduzierbar positioniert sind.

Die oben beschriebenen Merkmale lassen sich im wesentlichen beliebig miteinander kombinieren, so daß ein Multifunktionsinstrument entsteht.

### Bezugszeichenliste

- 10: Handgriff
- 11: Schraubgewinde
- 12: Federsitz
- 20: Druckleitung
- 20': Endabschnitt
- 20": Zuführungsabschnitt
- 21: Düse
- 22: Leitung
- 23: Sonde
- 24: Elektrode
- 25: Halter
- 30: Formteil
- 31: distales Ende
- 32: Endstück
- 33: Saugöffnung
- 34: Justieranschlag
- 35: Feder
- 36: Zentrierwendel
- 37: Überwurfkappe
- 38: Rohr
- 39: Halte
- 40: Lumen
- 41: HF-Anschluß
- 42: Isolierschicht
- 50: HF-Generator
- 51: APC-Generator
- P: Druckquelle
- E: Saugeinrichtung

## Patentansprüche

1. Applikator für die Wasserstrahl-Chirurgie, umfassend
einen Handgriff (10),
eine Druckleitung (20) zur Zuführung eines Arbeitsfluids aus einer Druckquelle (P),
eine Düse (21) am Ende der Druckleitung (20) zur Erzeugung eines Fluidstrahls mit definierten Eigenschaften,
**gekennzeichnet durch**
ein Set von Formteilen (30) mit voneinander verschiedenen Größen und/oder Formen, die mit dem Handgriff (10) reversibel verbindbar sind und Führungseinrichtungen (40, 34) aufweisen, um die elastisch verformbar ausgebildete Druckleitung (20) vom Handgriff (10) bis zur Düse (21) in einem **durch** das jeweilige Formteil (30) vorbestimmten Verlauf zu führen.

2. Applikator nach Anspruch 1,
**dadurch gekennzeichnet, daß**
die Führungseinrichtungen (40) derart ausgebildet sind, daß die am Handgriff (10) befestigte Druckleitung (20) bei einem Wechseln des Formteils (30) aus den Führungseinrichtungen (40, 34) herausnehmbar und wieder einsetzbar ist.

3. Applikator nach einem der vorhergehenden Ansprüche,
**dadurch gekennzeichnet, daß**
die Führungseinrichtungen (30) ein Lumen (40) in den rohrförmig ausgebildeten Formteilen (30) umfassen.

4. Applikator nach Anspruch 3,
**dadurch gekennzeichnet, daß**
das Lumen (40) der Formteile (30) an eine Saugeinrichtung anschließbar ist.

5. Applikator nach einem der vorhergehenden Ansprüche,
**dadurch gekennzeichnet, daß**
das Set mindestens ein geradliniges Formteil (30-1; 30-2) und ein gekrümmtes oder abgewinkeltes Formteil (30-3 bis 30-5) umfaßt.

6. Applikator nach einem der vorhergehenden Ansprüche,
**dadurch gekennzeichnet, daß**
das Formteil (30) mindestens an seinem vom Handgriff (10) abgewandten distalen Ende Elektrodeneintichtungen (32) aufweist, die mit einem HF-chirurgischen Generator (50, 51) zur Koagulation von Gewebe verbindbar sind.

7. Applikator nach einem der vorhergehenden Ansprüche,
**dadurch gekennzeichnet, daß**
das Formteil (30) eine vorzugsweise bis zur Düse (21) reichende Leitung (22/40) zum Zu- und/oder Abführen von Fluiden aufweist.

8. Applikator nach Anspruch 7,
**dadurch gekennzeichnet, daß**
die Leitung (22) mindestens eine Saugöffnung (33) aufweist, welche vorzugsweise in einer von der durch die Düse (21) vorgegebenen Richtung abweicht.

9. Applikator nach Anspruch 6,
**dadurch gekennzeichnet, daß**
das Formteil (30) eine Einrichtung, insbesondere eine schlauchförmige Sonde (23) zur Zuführung von Edelgas sowie eine vorzugsweise in der Sonde (23) angebrachte Elektrodeneinrichtung (24) zur Zuführung eines HF-Stromes zur Bildung einer Edelgas-Plasma-Koagulationseinrichtung umfaßt.

10. Applikator nach einem der vorhergehenden Ansprüche,
**dadurch gekennzeichnet, daß**
die Druckleitung (20) mit Düse (21) zusammen mit dem Handgriff (10) zur einmaligen Verwendung bestimmt und das Formteil (30) sterilisierbar ausgebildet sind.

11. Applikator nach einem der vorhergehenden Ansprüche,
**dadurch gekennzeichnet, daß**
das Formteil (30) und/oder die Druckleitung (20) eine Justiereinrichtung (34, 36) umfassen, die derart ausgebildet ist, daß die Düse (21) in einer definierten Position zum Formteil (30) positionierbar ist.

12. Applikator nach Anspruch 11,
**dadurch gekennzeichnet, daß**
die Justiereinrichtung eine Andrückeinrichtung (35) umfaßt zum Andrücken der Druckleitung (20) in Richtung des Fluidstrahls an einen am Formteil ausgebildeten Zentrieranschlag (34).

13. Applikator nach Anspruch 12,
**dadurch gekennzeichnet, daß**
die Druckleitung (20) einen, die Düse (21) beinhaltenden, verformbaren Endabschnitt (20') aufweist, der mit einem zur Druckquelle (P) führenden Zuführungsabschnitt (20") über eine Kupplung (25) verbindbar ist.

14. Applikator nach Anspruch 13,
**dadurch gekennzeichnet, daß**
der Endabschnitt (20) zusammen mit dem Formteil (30) austauschbar ist.

## Claims

1. Applicator for water-jet surgery, comprising
a handle (10),
a pressure conduit (20) to supply a working fluid from a pressure source (P),
a nozzle (21) at the end of the pressure conduit (20) to generate a fluid jet with specified properties,
**characterized by**
a set of molded parts (30) that differ from one another in size and/or shape, can be reversibly connected to the handle (10) and comprise guide devices (40, 34) with which to guide the elastically deformable pressure conduit (20) from the handle (10) to the nozzle (21) along a course predetermined by the relevant molded part (30).

2. Applicator according to Claim 1,
**characterized in that** the guide devices (40) are constructed in such a way that the pressure conduit (20) fixed to the handle (10) can be removed from and reinserted into the guide devices (40, 34) when the molded part (30) is being exchanged.

3. Applicator according to one of the preceding claims,
**characterized in that** the guide devices (30) consist of a lumen (40) in the tubular molded parts (30).

4. Applicator according to Claim 3,
**characterized in that** the lumen (40) of the molded parts (30) can be connected to a suction device.

5. Applicator according to one of the preceding claims,
**characterized in that** the set comprises at least one straight molded part (30-1; 30-2) and one curved or angled molded part (30-3 to 30-5).

6. Applicator according to one of the preceding claims,
**characterized in that** the molded part (30) comprises, at least at its distal end directed away from the handle (10), electrode devices (32) that can be connected to a HF surgical generator (50, 51) for the coagulation of tissue.

7. Applicator according to one of the preceding claims,
**characterized in that** the molded part (30) comprises a conduit (22/40) that preferably extends to the nozzle (21) and serves to supply or remove fluids.

8. Applicator according to Claim 7,
**characterized in that** the conduit (22) comprises at least one suction opening (33), which preferably is deflected in a direction predetermined by the nozzle (21).

9. Applicator according to Claim 6,
**characterized in that** the molded part (30) comprises a device, in particular a tubular probe (23), to supply noble gas as well as an electrode device (24), preferably disposed within the probe (23), to supply a HF current in order to form a noble-gas-plasma coagulation apparatus.

10. Applicator according to one of the preceding claims,
**characterized in that** the pressure conduit (20) with nozzle (21) together with the handle (10) is intended to be used only once, and the molded part (30) is designed to be sterilizable.

11. Applicator according to one of the preceding claims,
**characterized in that** the molded part (30) and/or the pressure conduit (20) comprise an adjustment device (34, 36) so constructed that the nozzle (21) can be placed in a specified position with respect to the molded part (30).

12. Applicator according to Claim 11,
**characterized in that** the adjustment device comprises a pressing device (35) to push the pressure conduit (20) in the direction of the fluid jet, against a centering stop (34) formed on the molded part.

13. Applicator according to Claim 12,
**characterized in that** the pressure conduit (20) comprises a deformable end section (20) that contains the nozzle (21) and can be connected by way of a coupling (25) to a supply section (20") that leads to the pressure source (P).

14. Applicator according to Claim 13,
**characterized in that** the end section (20') can be exchanged together with the molded part (30).

## Revendications

1. Applicateur pour la chirurgie à jet d'eau, comprenant
une poignée (10),
une conduite sous pression (20) pour alimenter un fluide de travail à partir d'une source de pression (P),
une buse (21) à l'extrémité de la conduite sous pression (20) pour générer un jet de fluide ayant des propriétés définies,
**caractérisé par**
un jeu de pièces moulées (30), ayant des tailles et/ou des formes différentes les unes des autres, qui peuvent être reliées de manière réversible à la poignée (10) et qui présentent des dispositifs de guidage (40, 34) permettant de guider la conduite sous pression (20), réalisée de manière déformable élastiquement, depuis la poignée (10) jusqu'à la buse (21) selon un profil prédéterminé par la pièce moulée (30) respective.

2. Applicateur selon la revendication 1, **caractérisé en ce que** les dispositifs de guidage (40) sont réalisés de telle façon que la conduite sous pression (20) fixée à la poignée (10) peut être retirée et remise en place dans les dispositifs de guidage (40, 34) lors d'un changement de la pièce moulée (30).

3. Applicateur selon l'une des revendications précédentes, **caractérisé en ce que** les dispositifs de guidage (30) comprennent une lumière (40) dans les pièces moulées (30) de forme tubulaire.

4. Applicateur selon la revendication 3, **caractérisé en ce que** la lumière (40) des pièces moulées (30) peut être reliée à un dispositif d'aspiration.

5. Applicateur selon l'une des revendications précédentes, **caractérisé en ce que** le jeu comprend au moins une pièce moulée rectiligne (30-1 ; 30-2) et une pièce moulée incurvée ou coudée (30-3 à 30-5).

6. Applicateur selon l'une des revendications précédentes, **caractérisé en ce que** la pièce moulée (30) présente au moins à son extrémité distale à l'opposé de la poignée (10) des dispositifs à électrodes (32) qui peuvent être reliés à un générateur HF chirurgical (50, 51) pour la coagulation tissulaire.

7. Applicateur selon l'une des revendications précédentes, **caractérisé en ce que** la pièce moulée (30) présente un conduit (22/40) s'étendant de préférence jusqu'à la buse (21) pour amener et/ou évacuer des fluides.

8. Applicateur selon la revendication 7, **caractérisé en ce que** le conduit (22) présente au moins un orifice d'aspiration (33) qui s'écarte de préférence d'une direction prédéterminée par la buse (21).

9. Applicateur selon la revendication 6, **caractérisé en ce que** la pièce moulée (30) comprend un dispositif, en particulier une sonde tubulaire (23), pour amener un gaz noble, ainsi qu'un dispositif à électrodes (24), de préférence placé dans la sonde (23), servant à amener un courant HF afin de former un système de coagulation par plasma de gaz noble.

10. Applicateur selon l'une des revendications précédentes, **caractérisé en ce que** la conduite sous pression (20) avec la buse (21) ainsi que la poignée (10) sont destinées à une utilisation unique et **en ce que** la pièce moulée (30) est conçue de façon à pouvoir être stérilisée.

11. Applicateur selon l'une des revendications précédentes, **caractérisé en ce que** la pièce moulée (30) et/ou la conduite sous pression (20) comprennent un système d'ajustement (34, 36) configuré de façon à permettre le positionnement de la buse (21) dans une position définie par rapport à la pièce moulée (30).

12. Applicateur selon la revendication 11, **caractérisé en ce que** le système d'ajustement comprend un dispositif de compression (35) servant à comprimer la conduite sous pression (20) en direction du jet de fluide contre une butée de centrage (34) formée sur la pièce moulée.

13. Applicateur selon la revendication 12, **caractérisé en ce que** la conduite sous pression (20) présente un segment d'extrémité (20'), contenant la buse (21) et déformable, qui peut être relié par le biais d'un raccord (25) à un segment d'amenée (20") conduisant à la source de pression (P).

14. Applicateur selon la revendication 13, **caractérisé en ce que** le segment d'extrémité (20') est interchangeable ensemble avec la pièce moulée (30).
